(19) Europäisches Patentamt
European Patent Office
Office européen des brevets

(11) **EP 2 582 300 B1**

(12) **EUROPÄISCHE PATENTSCHRIFT**

(45) Veröffentlichungstag und Bekanntmachung des Hinweises auf die Patenterteilung:
**08.05.2019 Patentblatt 2019/19**

(21) Anmeldenummer: **11737906.5**

(22) Anmeldetag: **15.06.2011**

(51) Int Cl.:
*A61B 6/03* (2006.01)   *G01N 23/04* (2018.01)
*G01N 23/20* (2018.01)   *A61B 6/00* (2006.01)
*G02B 5/18* (2006.01)   *G01N 23/00* (2006.01)

(86) Internationale Anmeldenummer:
**PCT/EP2011/059940**

(87) Internationale Veröffentlichungsnummer:
**WO 2011/157749 (22.12.2011 Gazette 2011/51)**

(54) **GENEIGTE PHASENGITTERSTRUKTUREN**

INCLINED PHASE GRATING STRUCTURES

STRUCTURES DE RÉSEAU DE PHASE INCLINÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.06.2010 DE 102010017425**

(43) Veröffentlichungstag der Anmeldung:
**24.04.2013 Patentblatt 2013/17**

(73) Patentinhaber: **Karlsruher Institut für Technologie**
**76131 Karlsruhe (DE)**

(72) Erfinder:
• **MOHR, Jürgen**
**75056 Sulzfeld (DE)**
• **PFEIFFER, Franz**
**85748 Garching (DE)**
• **REZNIKOVA, Elena**
**630087 Novosibirsk (DE)**
• **NAZMOV, Vladimir**
**76351 Linkenheim (DE)**

(74) Vertreter: **Fitzner, Uwe et al**
**Meissner Bolte**
**Patentanwälte Rechtsanwälte**
**Partnerschaft mbB**
**Hauser Ring 10**
**40878 Ratingen (DE)**

(56) Entgegenhaltungen:
WO-A1-2010/146498   WO-A1-2011/070489
WO-A1-2011/096584   US-A1- 2007 183 583

EP 2 582 300 B1

**Beschreibung**

Die vorliegende Erfindung betrifft Verbesserungen der Phasengitter in Anwendungen basierend auf dem Talbot-Effekt.

Stand der Technik:

Die Phasenkontrastbildgebung mit Röntgenstrahlen beruht auf einem gitterbasierten Interferometer, das den Talbot-Effekt zur Bildgebung nutzt. Dazu werden zwei Gitter senkrecht zum Röntgenstrahl parallel zueinander positioniert. Das Phasengitter $g_1$ besteht aus Linien, die einen Phasenschub von Pi (oder Pi/2) (Pi = 3.14159...) und eine vernachlässigbare Röntgenabsorption verursachen. Es wirkt als Strahlteiler und teilt die Röntgenstrahlen in die +1 und -1 Beugungsordnung. Die gebeugten Strahlen interferieren gemäß dem (fraktionalen) Talbot-Effekt und es entsteht ein Interferenzmuster, das in den zu den Gitterlinien senkrechten Richtungen periodisch ist. Das Interferenzbild hat eine maximale Modulation bei den fraktionalen Talbot-Abständen $d_m$: Für eine Parallelstrahlgeometrie beträgt $d_m$

$$d_m = m \frac{p_1^2}{8\lambda}$$

mit

$\lambda$ (Lambda) = Wellenlänge,
$p_1$ = Gitterperiode von $g_1$ (Phasengitter),
$m$ = ungerade Ganzzahl = Ordnung des fraktionalen Talbot-Abstandes Für die übliche Kegelstrahlgeometrie ändert sich $d_m$ um den Faktor

$$\frac{l}{l \quad d_m}$$

wobei I Abstand des Phasengitters von der Röntgenquelle ist.

Um die Position des Interferenzmusters zu bestimmen, wird dies mit dem Analysatorgitter $g_2$ abgetastet, das stark absorbierende Stege mit der Periode $p_2$ aufweist.

Für die Kegelstrahlgeometrie ergibt sich $p_2$ aus

$$p_2 = \frac{l}{l \quad d_M} \frac{p_1}{A}$$

mit A = 1 für Pi/2 und A = 2 für Pi Phasenverschiebung (Pi = 3.14159...). Damit ist das Verhältnis der Perioden $p_1$ und $p_2$ abhängig von der Wellenlänge, vom gewählten fraktionalen Talbot-Abstand und vom Abstand I des Phasengitters von der Röntgenquelle.

Da Abweichungen vom idealen Periodenverhältnis zu einer Verschlechterung der Bildqualität führen, muss für jedes Experiment ein in der Periode angepasster Gittersatz gewählt werden. Da die Herstellung der Gitter mit mikrotechnischen Methoden sehr aufwändig ist, ist dies sehr kostenintensiv. Außerdem besteht für den Anwender nicht die Möglichkeit mit einem vorgegebenen Gittersatz Messungen in unterschiedlichen Talbot-Abständen durchzuführen und so den Messaufbau an unterschiedliche Sensitivitäten der zu vermessenden Objekte anzupassen. Ein weiteres Problem besteht darin, dass die Periode je nach Herstellungsverfahren nur auf wenige Nanometer genau eingehalten werden kann. Beispielsweise ist die Positionsgenauigkeit eines Elektronenstrahlschreibers, der üblicherweise für die Erzeugung der primären Gitterstruktur eingesetzt wird, auf wenige Nanometer spezifiziert. Insofern ist insbesondere bei Gittern, für die jeweils eine Elektronenstrahllithographie notwendig ist, mit Schwankungen in der Periodenlänge zwischen Phasengitter und Analysatorgitter von wenigen Nanometern zu rechnen.

Genauere Angaben zu den beschriebenen Effekten und Apparaturen sind dem Fachmann hinreichend bekannt und müssen hier nicht näher beschrieben werden.

Aus US 2007/0183579 A1 und US 2009/0092227 A1 ist es bekannt, Phasengitter um die optische Achse entsprechend der Einfallsrichtung der Strahlen zu drehen, um dadurch störenden Moiréemustern zu begegnen.

DE 100 25 694 A1 betrifft Littrow-Konfigurationen unter Verwendung von UV-Strahlen (schwachen UV-Strahlen).

US 2009/0207416 A1 beschreibt Apparaturen basierend ausschließlich auf Licht (ggf. UV und IR), jedoch nicht auf Röntgenstrahlen.

In DE 10 2008 048 668 A1 wird versucht durch die Anordnung mehrerer Gitter den Effekt des Auffächerns des Röntgenstrahls zu umgehen, d.h. durch die Anordnung mehrerer Gitter nebeneinander soll erreicht werden, dass die Röntgenstrahlen gerade durch die Gitterstruktur passieren, da diese Gitterstrukturen senkrecht zu den jeweils einfallenden Röntgenstrahlen stehen.

Aufgabe:

Aufgabe der vorliegenden Erfindung ist es die Nachteile des Standes der Technik zu vermeiden.

Insbesondere sollen die Bildqualität, z.B. Phasenkontrast und Schärfe, mit einfachen Mitteln verbessert werden, Interferometer sollen variabler einsetzbar sein, so dass nicht für jedes Experiment ein eigenes Phasengitter verwendet werden muss und ökonomische Vorteile resultieren.

Lösung:

Diese Aufgabe wird durch spezielle Gitter bzw. Gitteranordnungen für die Phasengitter von Interferometern, sowie entsprechende Interferometer und Verfahren, sowie Verwendungen gelöst, bei denen die Gitterstruktur in Relation zu der Einstrahlungsrichtung bzw. der optischen Achse geneigt ist, entsprechend
einer Rotation des Gitters um die parallel zu den Gitterstreben laufende Gitterzentralachse oder
einer Rotation der einzelnen Gitterstreben um ihre jeweilige, parallel zu der Gitterzentralachse angeordnete Längsachse.

Begriffsdefinitionen:

Die Begriffe "Interferometer" bzw. "Gitterinterferometer" betreffen im Rahmen der vorliegenden Erfindung solche Geräte, die unter Ausnutzung des Talbot-Effektes arbeiten.

Detaillierte Beschreibung:

In der folgenden Beschreibung und in den folgenden Zeichnungen sind gleiche Teile beziehungsweise Merkmale in Beschreibung und den Zeichnungen durch die gleichen Ziffern bezeichnet. Die Zeichnungen sind nicht unbedingt maßstabsgetreu. Aus Gründen der Klarheit und zur einfacheren Darstellung können einige Merkmale der Erfindung übertrieben groß oder in schematischer Form dargestellt sein, ebenso können demgemäß einige Details von konventionellen bzw. bekannten Elementen nicht dargestellt sein.
Gegenstand der vorliegenden Erfindung sind Gitterinterferometer mit geneigten Phasengitterstrukturen.
Insbesondere werden die geneigten Phasengitterstrukturen durch

a) Neigung der Gitterstrukturen im Interferometeraufbau gegenüber der Einstrahlungsrichtung bzw. optischen Achse, entsprechend
einer Rotation des Gitters um die parallel zu den Gitterstreben laufende Gitterzentralachse, und/oder
b) Herstellung der Gitterstrukturen geneigt auf dem Substrat des Phasengitters, entsprechend einer Rotation der einzelnen Gitterstreben um ihre jeweilige, parallel zu der Gitterzentralachse angeordnete Längsachse.

erhalten.
Die vorliegende Erfindung umfasst demgemäß in drei alternativen Ausgestaltungen a), b) und a) + b).
Unter Punkt a) ist dabei bevorzugt entweder

- eine nachträgliche gewollte Neigung nach dem Einbau oder
- eine gewollte Neigung beim Einbau,

um einen bestimmten Neigungswinkel des Phasengitters bezogen zur Einstrahlungsrichtung bzw. zur optischen Achse zu erhalten, zu verstehen.
In einer bevorzugten Ausgestaltung der vorliegenden Erfindung ist eine Neigung der Gitterstrukturen im Interferometeraufbau gegenüber der Einstrahlungsrichtung bzw. optischen Achse durch versehentliche Dejustierung nicht gemeint und nicht umfasst. Die vorliegende Erfindung betrifft in dieser Ausgestaltung bewusst herbeigeführte Neigungen der Gitter durch Neigung gegenüber dem Ist-Zustand (z.B. Auslieferungszustand) des Interferometers.
Gegenstand der vorliegenden Erfindung ist demgemäß auch die Verwendung geneigter Gitterstrukturen als Phasengitter in der Interferometrie.
Weiterhin ist Gegenstand der vorliegenden Erfindung ein Verfahren zur Erhöhung der Bildqualität, insbesondere der Schärfe, von Phasenkontrastbildern bei Interferometern, bzw. bei Anwendungen die auf dem Talbot-Effekt beruhen, dadurch gekennzeichnet, dass als Phasengitter

i) Phasengitter, die im Interferometeraufbau gegenüber der Einstrahlungsrichtung bzw. optischen Achse geneigt sind, entsprechend einer Rotation des Gitters um die parallel zu den Gitterstreben laufende Gitterzentralachse, und/oder
ii) Phasengitter, bei denen die Gitterstrukturen geneigt auf dem Substrat des Phasengitters angeordnet sind, entsprechend einer Rotation der einzelnen Gitterstreben um ihre jeweilige, parallel zu der Gitterzentralachse angeordnete Längsachse

eingesetzt werden.
Die vorliegende Erfindung umfasst demgemäß in drei alternativen Ausgestaltungen i), ii) und i) + ii).
Ebenfalls beschrieben sind Phasengitter, bei denen die Gitterstrukturen geneigt auf dem Substrat des Phasengitters angeordnet sind.
Nicht zuletzt Gegenstand der vorliegenden Erfindung sind die Verwendungen des Interferometers und Verfahren.
Die vorliegende Erfindung betrifft insbesondere Gitterinterferometer an Synchrotronstrahlrohren bzw. für Röntgenstrahlung allgemein.
Bei Aufbau von Gitterinterferometern an Synchrotronstrahlrohren liegen die Unterschiede in den Gitterperioden im Bereich von wenigen Nanometern, typischerweise im Bereich von 2 nm bis 10 nm (also im Toleranzbereich des Fertigungsverfahrens), was bei Perioden von wenigen Mikrometern weniger als 1% der Periodenlänge entspricht. Bei Anwendungen mit Röntgenröhren z.B. in der Computertomographie liegen die Periodenänderungen im Bereich einiger 10 nm also im Bereich von einigen Prozent der Periodenlänge.
Periodenänderungen im genannten Bereich lassen sich erfindungsgemäß durch die Schrägstellung des Phasengitters relativ zum Strahl, entsprechend einer Rotation des Gitters um die parallel zu den Gitterstreben laufende Gitterzentralachse, erreichen. Hierbei ist die Periodenänderung proportional zu dem Winkel der Schrägstellung

$$p' = (1-\cos\Theta)*p, \Delta p = p*\cos\Theta$$

Beispielsweise erfordert eine Periodenänderung von 5 nm bei einer Periodenlänge von 2,5 μm eine Schrägstellung von ca. 3,63°.

Änderungen des Winkels bei Änderung des Talbot-Abstandes oder der Wellenlänge liegen eher im Bereich von Zehntelgrad und damit im Bereich unter 1 nm.

Derartige Änderungen in der Periode, aber auch bereits Änderungen im Bereich von wenigen Nanometern, liegen wie oben dargestellt bereits in der Toleranz des Herstellungsverfahrens. Die vorliegende Erfindung kann demgemäß also dazu genutzt werden, um die Fertigungstoleranzen bei der Herstellung auszugleichen, also als Fehlerkorrektur genutzt werden.

Für Periodenänderungen von z.B. 50 nm (Anwendung mit Röntgenröhren) und Perioden von 2,5 μm beträgt der Schrägstellungswinkel ca. 12°.

Eine erste erfindungsgemäße Variante i) besteht darin, die Gitterstrukturen senkrecht auf einem Substrat herzustellen, also die übliche Herstellung üblicher Phasengitter, und dann die Gitterstruktur im Interferometeraufbau um den gewünschten Winkel zu neigen, entsprechend einer Rotation des Gitters um die parallel zu den Gitterstreben laufende Gitterzentralachse.

Bei dieser Variante stehen dann die Strukturen geneigt zum Strahl und erzeugen dabei kein rechteckiges Absorptionsprofil.

Je nach verwendetem Material und im Experiment verwendeter Energie kann die Höhe der Lamellenstrukturen der Phasengitter deutlich über 50 μm betragen (z.B. bei Verwendung von Silizium oder Kunststoff). In diesem Falle kann es bereits bei Winkeln um 1° zu Überdeckungen der Lamellen kommen, so dass das Intensitätsprofil nicht mehr eindeutig ist. Im Falle der Verwendung von hochbrechenden Materialien als Phasengitter (z.B. Metallen) liegt die Höhe im Bereich von wenigen Mikrometern, so dass z.B. bei 10 μm Höhe Winkeländerungen in Bezug zur senkrechten Orientierung von bis ca. 5° möglich sind, ohne dass es zu Überdeckungen kommt.

Bei dieser Variante i) ist es demgemäß bevorzugt, Metalle als Phasengitter einzusetzen.

Dabei können alle Metalle eingesetzt werden, die galvanisch abscheidbar sind, insbesondere wenn die Gitter über LIGA-Verfahren hergestellt werden.

Es ist bevorzugt, Metalle ausgewählt aus der Gruppe bestehend aus Nickel, Kupfer, Gold, Wolfram, Mischungen, Legierungen und Kombinationen daraus, einzusetzen. Besonders bevorzugt sind Kupfer und Nickel, insbesondere Nickel als Metalle für die Phasengitter.

Bei dieser Variante ist demgemäß besonders darauf zu achten, dass die Winkeländerung an die jeweiligen Gegebenheiten anzupassen ist; dies kann gegebenenfalls durch einfache Vorversuche erfolgen.

Eine zweite erfindungsgemäße Variante ii) besteht darin, die Strukturen mit dem für den üblichen bzw. jeweiligen Aufbau notwendigen Winkel (Periodenlänge) von wenigen Grad auf dem Substrat herzustellen, entsprechend einer Rotation der einzelnen Gitterstreben um ihre jeweilige, parallel zu der Gitterzentralachse angeordnete Längsachse, und das Substrat mit diesem Winkel in den Strahl einzubauen.

In diesem Fall werden die Strukturen gerade durchstrahlt. Da die Winkeländerungen bei Änderung des Talbot-Abstandes gering sind und eher im Bereich kleiner 1° liegen, können diese Änderungen ausgehend von dem bereits voreingestellten Winkel mit nur geringer Qualitätseinbuße in den Bildern realisiert werden. Es kommt auch bei sehr hohen Lamellenstrukturen zu keiner Überdeckung und die Flankensteilheit im Absorptionsprofil bleibt hoch. Diese Vorgehensweise ist bei hohen Röntgenenergien auch bei Metallgittern von Vorteil und erlaubt die Anwendung der Methode auf praktische alle Röntgenenergien.

Durch diese Variante ii) lässt sich das bei der ersten Variante möglicherweise auftretende Problem der Überdeckung vermeiden.

In einer Ausführungsform der vorliegenden Erfindung ist diese zweite Variante ii) demgemäß bevorzugt.

Es ist ebenfalls Teil der vorliegenden Erfindung, die Varianten i) und ii) zu kombinieren.

Die Herstellung der Phasenkontrastgitter weist dabei keine Besonderheiten auf und erfolgt nach üblichen Verfahren, wie z.B. dem LIGA-Verfahren, per UV-Lithographie oder per Elektronenstrahlschreiber, wobei UV-Lithographie und Elektronenstrahlschreibung für höhere Strukturen (höher 2 - 3 μm) weniger geeignet sind.

Phasengitter, bei denen die Gitterstrukturen auf dem Träger nicht geneigt sind, sind auch über Trockenätzverfahren (z.B. DRIE) oder Laserablation herstellbar.

Die vorliegende Erfindung umfasst demgemäß Interferometer, bei denen die Phasengitter, entweder solche üblichen Aufbaus oder solche mit geneigten Lamellenstrukturen, von außen regelbar bzw. verstellbar sind, d.h. ohne das das Interferometer geöffnet werden muss.

Die Regelung bzw. Verstellung erfolgt dabei rein mechanisch, z.B. über Drehregler, oder automatisch rechner- bzw. computergestützt.

Insbesondere bei der computergestützten Ausführungsvariante kann während die Messung läuft die Neigung des Phasengitters sehr fein reguliert werden, so dass eine optimale Abbildungsleistung erzielt werden kann.

Die Regelung erfindungsgemäßer Interferometer von außen kann demgemäß selbstverständlich auch in verschiedene Richtungen erfolgen.

In einer weiteren Variante der vorliegenden Erfindung sind Interferometer umfasst, die mehrere Phasengitter enthalten, die von außen gewechselt und geneigt werden können, d.h. ohne das das Interferometer geöffnet werden muss, z.B. über Drehknöpfe oder durch Computersteuerung. Die mehreren Phasengitter können z.B. in Art eines Revolvermagazins oder in Art eines Diaprojektormagazins angeordnet sein.

Auf diese Weise ist es möglich einen sehr großen Winkelbereich abzudecken und die Variabilität der Interferometer noch weiter zu steigern.

Ein erheblicher Vorteil der vorliegenden Erfindung besteht darin, dass die Strukturen für die beiden Gitter mit derselben Prozesstechnik (Lithographieverfahren) und damit ausgehend von derselben (mit dem Elektronenstrahlschreiber hergestellten) Primärstruktur bzw. der daraus resultierenden Mastermaske hergestellt werden können. Neben der Tatsache, dass damit die geforderten Periodenänderungen sehr exakt eingestellt werden können und nicht den Toleranzen in der Primärstrukturierung unterliegen, werden prozessbedingte Abweichungen von der Sollgeometrie identisch auf die beiden Gitterstrukturen (Phasengitter und Intensitätsgitter) übertragen.

Insofern werden Störeffekte durch die prozessbedingten Abweichungen minimiert bzw. sogar vollständig kompensiert.

Aus dem Stand der Technik sind keine ähnliche Lösungen bekannt. Bisher wurde jede Gitterstruktur ausgehend von einer in der Periodenlänge angepassten Primärstruktur gefertigt.

Demgemäß ist auch Gegenstand der vorliegenden Erfindung ein Verfahren zur Herstellung von Gitterstrukturen geneigt auf dem Substrat des Phasengitters, entsprechend einer Rotation der einzelnen Gitterstreben um ihre jeweilige, parallel zu der Gitterzentralachse angeordnete Längsachse, bei dem Phasengitter G1 und Analysengitter G2 für ein Röntgeninterferometer ausgehend von der gleichen Mastermaske hergestellt werden, insbesondere über Elektronenstrahllithographie (E-Beam-Lithographie), dadurch gekennzeichnet, dass

- eine Mastermaske eingesetzt wird, welche die Periode von dem Analysengitter G2 hat,
- das Analysengitter G2 durch direkte senkrechte Belichtung entsteht, und
- das Phasengitter G1, durch schräge Belichtung oder schräge Anordnung unter einem Winkel, durch Projektion mit der Gitterperiode G1 entsteht.

Das entscheidende Merkmal hierbei ist, dass die beiden Gitter von der gleichen Mastermaske hergestellt werden und damit die gleichen Prozessfehler haben. Durch Neigen des Phasengitters, erzielt man die Übereinstimmung der Gitterperioden, Fehler in der Herstellung der Gitter mitteln sich, zumindest weitgehend, heraus.

Die besonderen Vorteile dieses erfindungsgemäßen Verfahrens lassen sich dabei wie folgt darstellen: Bei einer Herstellungstoleranz der Gitter von z.B. 10 nm (Toleranz der E-Beam-Lithographie, die zur Herstellung der Gittermaske verwendet wird), kann es passieren, dass bei unabhängigen Gittern die Perioden absolut um 20 nm abweichen oder über die gesamte Gitterstruktur um 20 nm schwanken. Bei einer Periode von 2 $\mu$m würde dies bedeuten, dass ein Moireemuster mit einer Periode von 100 * 2 $\mu$m = 200 $\mu$m entsteht.

Wenn sich die Perioden der beiden Gitter aufgrund des fächerförmigen Röntgenstrahls um 0,1 $\mu$m unterscheiden müssen, so kann dies mit einer Schrägstellung von einem Winkel von 3 Grad erreicht werden. Da eine Winkeleinstellung auf 0,1 Grad kein Problem darstellt, kann man die Periode mit einer Genauigkeit auf 4 nm einstellen. Bei einer Genauigkeit von 0,01 Grad liegt die Periodengenauigkeit bei 0,3 nm.

Wenn die Gitter mit der gleichen E-Beam-Mastermaske hergestellt werden, kann man jedoch eine deutlich höhere Genauigkeit erhalten, als im Falle unabhängig hergestellter Gitter, so dass bei Einsatz von durch das erfindungsgemäße Verfahren hergestellten Gittern deutlich weniger Moireemuster entstehen und die Abbildungsleistung entsprechend besser ist.

Periodenschwankungen in den beiden Gittern, die mit derselben E-Beam-Mastermaske hergestellt wurden, spielen praktisch keine Rolle, da sie an der gleichen Stelle sind und damit größere Perioden in einer hell/dunkel-Schwankung an der Stelle mit größerer Gitterperiode detektiert werden. Für Gitter, die mit verschiedenen Masken hergestellt wurden, ist dies ein Problem.

Durch die vorliegende Erfindung ist es auch möglich, ein einziges Phasengitter für verschiedene Messungen zu verwenden.

Die vorliegende Erfindung stellt demgemäß eine erhebliche Verfahrenserleichterung dar. Ebenso können durch die vorliegende Erfindung erhebliche Kosteneinsparungen erhalten werden, weil weniger Phasengitter notwendig sind.

Ein Vorteil der vorliegenden Erfindung ist mit anderen Worten, dass die Phasenkontrastgitter durch die Neigung variable Perioden ermöglichen.

Die vorliegende Erfindung betrifft auch die Phasenkontrasttomographie mit Röntgenstrahlung.

Besonders vorteilhaft bei der vorliegenden Erfindung ist, dass die Phasenkontrastbilder schärfer werden und keine zusätzlichen Intensitätsmodulationen enthalten.

Die erfindungsgemäßen geneigten Gitter können verwendet werden in röntgenbasierten Systemen der Medizintechnik (z.B. Röntgentomographie) sowie in Geräten zur zerstörungsfreien Prüfung von Materialien und Komponenten.

Die erfindungsgemäßen Gitterinterferometer können in einer Variante der vorliegenden Erfindung verwendet werden in Gantry-Einheiten von Computertomographen, in Röntgenoptiken von Mikro-CT-Geräten, in röntgenoptischen Systemen für die medizinische Radiographie (Mammographie) bzw. Angiographie.

Die verschiedenen Ausgestaltungen der vorliegenden Erfindung, z.B. aber nicht ausschließlich diejenigen der verschiedenen abhängigen Ansprüche, können dabei in beliebiger Art und Weise miteinander kombiniert werden.

Figurenbeschreibung:

Bezugszeichenliste:

1 Maske (für die Herstellung)
2 Foto-Resist
3 Metall, bevorzugt Gold nach Galvanik
4 Substrat

Figur 1:
Die Figur illustriert eine Kippung bei senkrecht strukturierten Gittern. Sie zeigt ein Phasengitter, bei dem die Gitterstruktur senkrecht auf einem Substrat 4 hergestellt und dann die Gitterstruktur im Interferometeraufbau um den gewünschten Winkel geneigt wird.

Figur 2:

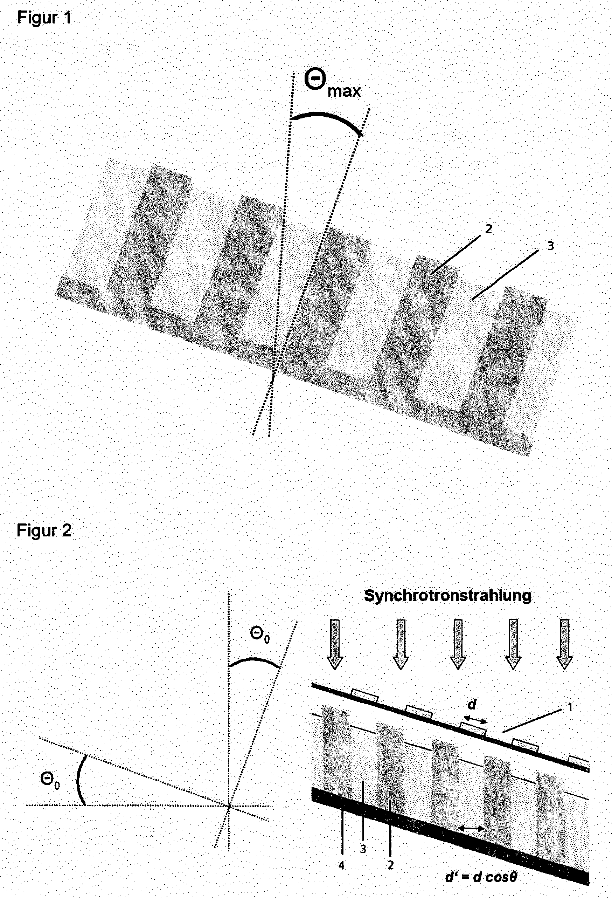

Figur 2 illustriert die zweite erfindungsgemäße Variante, bei der die Strukturen mit dem für den üblichen Aufbau notwendigen Winkel (Periodenlänge) von wenigen Grad auf dem Substrat 4 hergestellt werden und das Substrat mit diesem Winkel in den (Synchrotron-)Strahl eingebaut wird.
Dabei werden die Strukturen gerade durchstrahlt.

Figur 3:
Dies Figur illustriert, die Vorteile der zweiten erfindungsgemäßen Variante; da die Winkeländerungen bei Änderung des Talbot-Abstandes gering sind und eher im Bereich kleiner 1° liegen, können diese Änderungen ausgehend von dem bereits voreingestellten Winkel mit nur geringer Qualitätseinbuße in den Bildern realisiert werden. Es kommt auch bei sehr hohen Lamellenstrukturen zu keiner Überdeckung und die Flankensteilheit im Absorptionsprofil bleibt hoch.

Figur 4:
Phasenkontrast-Radiographieaufnahmen einer Münze bei 82 keV Röntgenenergie.
Links: Aufnahme mit nicht divergenzkompensierten Gittern (divergenzkompensiert bedeutet hier geneigt und auf die Situation, Periode angepasst). Rechts: Aufnahme mit fast vollständig divergenzkompensierten Gittern (hier konkret 5° Divergenzkompensation bei 155 m Quellabstand, Gitterperiode 2.4 $\mu$m,

55 cm Abstand zwischen den Gittern).

Es ist deutlich ersichtlich, dass die Phasenkontrastbilder schärfer werden und keine zusätzlichen Intensitätsmodulationen enthalten.

**Patentansprüche**

**1.** Gitterinterferometer, das unter Ausnutzung des Talbot-Effektes arbeitet, insbesondere für Röntgenstrahlung, mit geneigten Phasengitterstrukturen, wobei das Interferometer mindestens ein Phasengitter und ein Intensitätsgitter umfasst, wobei die Phasengitterstrukturen senkrecht auf einem Substrat des Phasengitters hergestellt und um einen bestimmten Neigungswinkel des Phasengitters gegenüber einer Einstrahlungsrichtung, entsprechend einer Rotation des jeweiligen Gitters um die parallel zu den Gitterstreben laufende Gitterzentralachse, geneigt sind, oder die Phasengitterstrukturen mit einem Winkel geneigt auf dem Substrat des Phasengitters, entsprechend einer Rotation der einzelnen Gitterstreben um ihre jeweilige, parallel zu der Gitterzentralachse angeordnete Längsachse, wobei die Gitterzentralachse parallel zu den Gitterstreben läuft, hergestellt sind und das Substrat mit diesem Winkel zur Einstrahlungsrichtung derart in den Strahl eingebaut ist, dass die Phasengitterstrukturen gerade durchstrahlt werden; wobei die Neigung des Phasengitters gegenüber einer Einstrahlungsrichtung von außen rein mechanisch oder rechner- bzw. computergestützt automatisch regelbar bzw. einstellbar ist.

**2.** Gitterinterferometer nach Anspruch 1, **dadurch gekennzeichnet, dass** mehrere austauschbare Phasengitter enthalten sind.

**3.** Verfahren zur Erhöhung der Bildqualität von Phasenkontrastbildern bei Interferometern bzw. bei Anwendungen die auf dem Talbot-Effekt beruhen durch ein Gitterinterferometer nach Anspruch 1 oder 2, **dadurch gekennzeichnet, dass**

i) ein Phasengitter im Interferometeraufbau gegenüber der Einstrahlungsrichtung geneigt ist, entsprechend einer Rotation des jeweiligen Gitters um die parallel zu den Gitterstreben laufende Gitterzentralachse, wobei Gitterstrukturen senkrecht auf einem Substrat des Phasengitters hergestellt sind, und/oder

ii) ein Phasengitter eingesetzt wird, bei dem die Gitterstrukturen geneigt auf dem Substrat des Phasengitters, entsprechend einer Rotation der einzelnen Gitterstreben um ihre jeweilige, parallel zu der Gitterzentralachse angeordnete Längsachse, wobei die Gitterzentralachse parallel zu den Gitterstreben läuft, angeordnet sind, wobei die Gitterstrukturen mit einem Winkel geneigt auf dem Substrat des Phasengitters hergestellt sind und das Substrat mit diesem Winkel zur Einstrahlungsrichtung derart in den Strahl eingebaut ist, dass die Phasengitterstrukturen gerade durchstrahlt werden.

4. Verfahren zur Herstellung von Gitterstrukturen geneigt auf dem Substrat des Phasengitters, entsprechend einer Rotation der einzelnen Gitterstreben um ihre jeweilige, parallel zu der Gitterzentralachse angeordnete Längsachse, bei dem Phasengitter und Analysengitter für ein Röntgeninterferometer ausgehend von der gleichen Mastermaske hergestellt werden, **dadurch gekennzeichnet, dass**

    - eine Mastermaske eingesetzt wird, welche die Periode von dem Analysengitter hat,
    - das Analysengitter durch direkte senkrechte Belichtung entsteht, und
    - das Phasengitter, durch schräge Belichtung oder schräge Anordnung unter einem Winkel, durch Projektion mit der Gitterperiode entsteht.

5. Verfahren nach Anspruch 4, **dadurch gekennzeichnet, dass** die Gitter mittels durch Elektronenstrahllitographie hergestellte Mastermasken hergestellt werden.

6. Verwendung eines Gitterinterferometers nach Anspruch 1 oder 2 in der Interferometrie, insbesondere für Röntgenstrahlung, unter Ausnutzung des Talbot-Effektes, insbesondere zur Erhöhung bzw. Verbesserung der Bildqualität, wobei die Gitterstrukturen senkrecht auf einem Substrat des Phasengitters hergestellt und um einen bestimmten Neigungswinkel des Phasengitters gegenüber einer Einstrahlungsrichtung, entsprechend einer Rotation des jeweiligen Gitters um die parallel zu den Gitterstreben laufende Gitterzentralachse, geneigt werden oder die Gitterstrukturen mit einem Winkel geneigt auf dem Substrat des Phasengitters, entsprechend einer Rotation der einzelnen Gitterstreben um ihre jeweilige, parallel zu der Gitterzentralachse angeordnete Längsachse, wobei die Gitterzentralachse parallel zu den Gitterstreben läuft, hergestellt sind und das Substrat mit diesem Winkel zur Einstrahlungsrichtung derart in den Strahl eingebaut wird, dass die Phasengitterstrukturen gerade durchstrahlt werden.

7. Verwendung eines Gitterinterferometers nach Anspruch 1 oder 2 in röntgenbasierten Systemen der Medizintechnik, in Geräten zur zerstörungsfreien Prüfung von Materialien und Komponenten, in Gantry-Einheiten von Computertomographen, in Röntgenoptiken von Mikro-CT-Geräten, in röntgenoptischen Systemen für die medizinische Radiographie (Mammographie) bzw. Angiographie

**Claims**

1. A grating interferometer working by use of the Talbot effect, in particular for radiation, with inclined phase grating structures, wherein the interferometer has at least a phase grating and an intensity grating, wherein the phase grating structures are produced perpendicularly on a substrate of the phase grating and are inclined at a specific inclination angle of the phase grating in relation to the irradiation direction, corresponding to a rotation of the respective grating about the grating central axis which runs parallel to the grating ribs, or the phase grating structures are produced at an angle inclined on the substrate of the phase grating, corresponding to a rotation of the individual grating ribs about their respective longitudinal axis which is arranged parallel to the grating central axis and the substrate at this angle with relation to the irradiation direction is installed into the beam in such a manner that the radiation passes through the phase grating structures in a straight line; whereby the inclination of the phase grating in relation to the irradiation direction can be regulated or set purely mechanically or computer-controlled from the outside.

2. The grating interferometer as claimed in claim 1, **characterized in that** it contains a plurality of interchangeable phase gratings.

3. A method for increasing the image quality of phase-contrast images in interferometers, or in applications which are based on the Talbot effect by a grating interferometer according to claim 1 or 2, **characterized in that**

    i) a phase grating in the interferometer set-up is inclined in relation to the irradiation direction, corresponding to a rotation of the respective grating about the grating central axis which runs parallel to the grating ribs, whereby the grating structures are produced perpendiularly on a substrate of the phase grating, and/or
    ii) use is made of a phase grating, in which the grating structures are installed at an incline on the substrate of the phase grating, corresponding to a rotation of the individual grating ribs about their respective longitudinal axis, which is arranged parallel to the grating central axis, whereby the grating central axis runs parallel to the grating ribs and the grating structures are produced at an angle inclined on the substrate of the phase grating and the substrate is installed into the beam at this angle in relation to the irradiation direction in such a manner that the radiation passes through the phase grating structures in a straight line.

4. Method for producing grating structures inclined on the substrate of the phase grating, corresponding to a rotation of the individual grating ribs about its respective longitudinal axis which is arranged parallel to the grating central axis.
wherein the phase grating and analysis grating for a ray interferometer are produced starting from the same master mask, **characterized in that**

   - use is made of a master mask which has the period of the analysis grating,
   - the analysis grating is created by direct perpendicular exposure, and
   - the phase grating is created by oblique exposure or oblique arrangement at an angle, by a projection with the grating period.

5. The method as claimed in claim 4, **characterized in that** the gratings are produced by means of master masks produced by means of electron beam lithography.

6. The use of a grating interferometer according to claim 1 or 2 in the interferiometry, in particular for radiation, by using the Talbot effect, in particular for increasing or improving the image quality wherein the grating structures are produced perpendicularly on the substrate of the phase grating, and are inclined at a specific inclination angle of the phase grating in relation to the irradiation direction corresponding to a rotation of the respective grating about the grating central axis which runs parallel to the grating ribs or
the grating structures are produced at an angle inclined on the substrate of the phase grating, corresponding to a rotation of the individual grating ribs about their respective longitudinal axis which runs parallel to the grating central axis and the substrate at this angle is installed into the beam in relation to the irradiation direction in such a manner that the radiation passes through the phase grating structures in a straight line.

7. The use of a grating interferometer according to claim 1 or 2 in ray-based systems in medical engineering, in instruments for nondestructive testing of materials and components, in gantry units of computer tomographs, I n X-ray optical units of micro-CT instruments, in ray optical systems for medical radiography (mammography) or a angiography.

**Revendications**

1. Un interféromètre à réseau utilisant l'effet Talbot, en particulier pour le rayonnement, avec des structures à réseau de phase inclinées,
où l'interféromètre a au moins un réseau de phase et un réseau d'intensité,
où les structures de réseau de phase sont produites perpendiculairement sur un substrat du réseau de phase et sont inclinées d'un angle spécifique du réseau de phase en relation à la direction d'irradiation correspondant à une rotation du réseau respectif autour d 'axe central qui s'étend parallèlement à les contre-fiches à réseau, ou
les structures à réseau de phase sont produites inclineés à une angle sur le substrat du réseau de phase correspondant à une rotation des contre-fiches à réseau individuelles autour de leur axe longitudinal respectif, que s'étend parallèlement à l'axe central, et l'axe central à réseau s'étend parallèlement à les contre-fiches, et le substrat est installé à cet angle en relation à la direction d'irradiation de telle manière que la radiation passe parles structures de réseau de phase en ligne droite;
où l' inclination du réseau de phase par rapport à la direction de l'irradiation peut être, de l'extérieur, ajustable et réglable mécaniquement ou automécaniquement assisté par ordinateur.

2. L'interféromètre selon la revendication 1, caractérisée in ce qu' une pluralité des réseaux de phase interchangeables est contenue.

3. Un procédé pour l'augmentation de la qualité des images de contraste de phase en des interféromètres, ou dans les applications basées sur l'effet Talbot par un interféromètre à réseau selon la revendication 1 ou 2, **caractérisée en ce**

   i) **qu'**un réseau de phase dans la contruction de l' interféromètre est incliné par relation à la direction d' irradiation, correspondant à la rotation du réseau respectif autour d'axe central que passe parallèlement au contre-fiches à réseau, et les structures à réseau sont produites perpendiculairement sur un substrat du réseau de phase, et/ou
   ii) un réseau de phase est installé, dans lequel les structures à réseau sont arrangées avec une inclination sur le substrat du réseau de phase, correspondant à une rotation des contre-fiches à réseau individuelles atour de leur axe longitudinal respectif, parallèlement à l'axe central de réseau, l'axe central passant parallèlement aux contre-fiches à réseau, et les structures à réseau sont produites inclinées avec un angle sur le substrat du réseau de phase et le substrat est intégré dans le rayon avec cet angle en relation à la direction d'irradiation de telle manière, que les structures à réseau de phase sont irradiées en ligne droite.

4. Procédé pour la production des structures à réseau inclinées sur le substrat du réseau de phase, correspondant à la rotation des contre-fîches individuelles autour de leur axe longitudinal lequel est arrangé parallèlement à l'axe central, et le réseau de phase et le réseau d' analyse sont produit pour un interféromètre à rayons, sortant de la même masque de maître, **caractérisé en ce que**

- une masque de maître est utilisée, quelle a la période du réseau d'analyse,
- le réseau d'analyse est créé par l'exposition perpendiculaire directe, et
- le réseau de phase est créé par l'exposition oblique ou disposition oblique à un angle, par projection avec la période de réseau.

5. Le procédé selon la revendication 4, **caractérisée en ce que** les réseaux sont produits par des moyens des masques de maîtres produites par des moyens de la lithographie par faisceau d'électrons.

6. Utilisation d'un interférométre de réseau selon la revendication 1 ou 2 en l'interfériometrie, particulièrement pour le rayonnement, en utilisant l'effet Talbot , en particulier pour l'augmentation et amélioration le qualité d' image **caractérisée en ce que** les structures de réseau sont produites perpendiculairement sur le substrat du réseau de phase, inclinées d'un angle spécifique du réseau de phase en relation à la direction d' irradiation correspondant à la rotation du réseau respectif autour de l '   axe central de réseau que passe parallèlement à les contre-fiches à reseau.

et les structures de réseau de phase sont produites inclinées à un angle sur le substrat du réseau de phase correspondant à une rotation des contre-fiches à réseau individuelles autour leurs l'axe longitudinal respectif, assemblée parallèlement à l'axe central, òu l'axe central passe parallèlement aux contre-fiches à réseau, et le substrat à cet angle et installé en relation à la direction d'irradiation dans le rayon en telle manière que la radiation passe par les structures à réseau de phase en ligne droite.

7. Utilisation d'un interféromètre selon la revendication 1 or 2 en des systèmes de la technologie médicale à base des rayons, dans les instruments et composants pour l'essai non-destructif , dans les unités Gantry des tomographes computérisés, dans les optiques à rayons des instruments de micro-CT, dans les systèmes optiques à rayons pour la radiographie médicale (mammographie) ou angiographie.

Figur 1

Figur 2

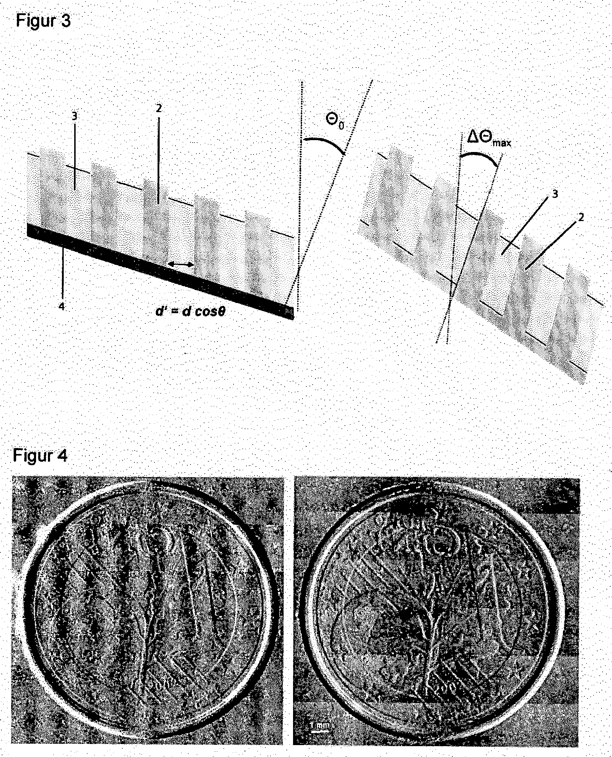

## Figur 3

## Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070183579 A1
- US 20090092227 A1
- DE 10025694 A1
- US 20090207416 A1
- DE 102008048668 A1